# EUROPEAN PATENT SPECIFICATION

(11) **EP 1 175 904 B1**
(45) Date of publication and mention of the grant of the patent: **28.03.2007**
(21) Application number: 01201913.9
(22) Date of filing: 17.07.1998
(51) Int. Cl.: A61K 31/663, A61P 19/10

(54) **Alendronate for use in the treatment of osteoporosis**
Alendronate zur Behandlung von Osteoporose
L'alendronate destiné à être utilisé dans le traitment de l'ostéoporose

(30) Priority: 22.07.1997 US 53351 P; 23.07.1997 US 53535 P; 20.08.1997 GB 9717590; 22.08.1997 GB 9717850
(43) Date of publication of application: 30.01.2002
(62) Divisional of application: 98935752.0
(73) Proprietor: Merck & Co. Inc., Rahway New Jersey 07065-0900 (US)
(72) Inventor: Daifotis, Anastasia G., Rahway, NJ 07065 (US); Santora, Arthur C., II, Rahway, NJ 07065 (US); Yates, John A., Rahway, NJ 07065 (US)
(74) Representative: Horgan, James Michael Frederic

(56) References cited:
- EP-A1- 0 274 158
- WO-A1-94/00129
- WO-A1-95/08331
- WO-A1-95/28936
- SINGER F R ET AL: "Bisphosphonates in the treatment of disorders of mineral metabolism." ADVANCES IN ENDOCRINOLOGY AND METABOLISM, (1995) 6 259-88. REF: 109 JOURNAL CODE: CB4. ISSN: 1049-6734., XP002092145 United States
- LIBERMAN U A ET AL: "EFFECT OF ORAL ALENDRONATE ON BONE MINERAL DENSITY AND THE INCIDENCE OF FRACTURES IN POSTMENOPAUSAL OSTEOPOROSIS" THE NEW ENGLAND JOURNAL OF MEDICINE, vol. 333, no. 22, 30 November 1995 (1995-11-30), pages 1437-1443, XP000579307
- BANKHURST A ET AL: "THREE-YEAR TREATMENT WITH ALENDRONATE PREVENTS FRACTURES IN WOMEN WITH POSTMENOPAUSAL OSTEOPOROSIS" ARTHRITIS AND RHEUMATISM, vol. 38, no. 9, SUPPL, 1 September 1995 (1995-09-01), page S359 XP000579368
- FILIPPONI P ET AL: "CYCLICAL CLODRONATE IS EFFECTIVE IN PREVENTING POSTMENOPAUSAL BONE LOSS: A COMPARATIVE STUDY WITH TRANSCUTANEOUS HORMONE REPLACEMENT THERAPY" JOURNAL OF BONE AND MINERAL RESEARCH, vol. 10, no. 5, May 1995 (1995-05), pages 697-703, XP002042531
- MCCLUNG M R ET AL: "Tiludronate therapy for Paget's disease of bone [published erratum appears in Bone 1996 Mar;18(3):292]." BONE, (1995 NOV) 17 (5 SUPPL) 493S-496S. JOURNAL CODE: ASR. ISSN: 8756-3282., XP002092146 United States
- SELTENMEYER, Y. ET AL: "A comparison of the antiresorptive potency of various bisphosphonates in vivo with their inhibitory effect in vitro on squalene synthase and cellular sterol synthesis." BONE (NEW YORK), (1997) VOL. 20, NO. 4 SUPPL., PP. 114S. MEETING INFO.: 25TH EUROPEAN SYMPOSIUM ON CALCIFIED TISSUES HARROGATE, ENGLAND, UK APRIL 25-29, 1997 ISSN: 8756-3282., XP002092147
- ADACHI J.D.: "osteoporosis-Its Diagnosis, Management and Treatment with New Oral Bisphophonate Agent, Etidronate" TODAY'S THERPAEUTIC TRENDS, vol. 14, no. 1, 1996, pages 13-24, XP002092148
- BELL, NORMAN H. ET AL: "Bisphosphonates in the treatment of osteoporosis" ENDOCRINE (1997), 6(2), 203-206 CODEN: EOCRE5;ISSN: 1355-008X, XP002092149
- "Update: Bisphosphonates" LUNAR NEWS, vol. April97, 1 April 1997 (1997-04-01),
- "Update: Bisphosphonates" LUNAR NEWS, vol. July96, 1 July 1996 (1996-07-01),

## Description

### BACKGROUND OF THE INVENTION

A variety of disorders in humans and other mammals involve or are associated with abnormal bone resorption. Such disorders include, but are not limited to, osteoporosis, Paget's disease, periprosthetic bone loss or osteolysis, and hypercalcemia of malignancy. The most common of these disorders is osteoporosis, which in its most frequent manifestation occurs in postmenopausal women. Osteoporosis is a systemic skeletal disease characterized by a low bone mass and microarchitectural deterioration of bone tissue, with a consequent increase in bone fragility and susceptibility to fracture. Because osteoporosis, as well as other disorders associated with bone loss, are chronic conditions, it is believed that appropriate therapy will generally require chronic treatment.

Multinucleated cells called osteoclasts are responsible for causing bone loss through a process known as bone resorption. It is well known that bisphosphonates are selective inhibitors of osteoclastic bone resorption, making these compounds important therapeutic agents in the treatment or prevention of a variety of generalized or localized bone disorders caused by or associated with abnormal bone resorption. *See* H. Fleisch, *Bisphosphonates In Bone Disease, From The Laboratory To The Patient,* 2nd Edition, Parthenon Publishing (1995).

At present, a great amount of preclinical and clinical data exists for the potent bisphosphonate compound alendronate. Evidence suggests that other bisphosphonates such as risedronate, tiludronate, ibandronate and zolendronate, have many properties in common with alendronate, including high potency as inhibitors of osteoclastic bone resorption. An older bisphosphonate compound, etidronate, also inhibits bone resorption. However, unlike the more potent bisphosphonates, etidronate impairs mineralization at doses used clinically, and may give rise to osteomalacia, a condition resulting in an undesirable decrease in bone mineralization. *See* Boyce, B. F., Fogelman, I., Ralston, S. et al. (1984) Lancet 1(8381), pp. 821-824 (1984), and Gibbs, C. J., Aaron, J. E.; Peacock, M. (1986) Br. Med. J. 292, pp. 1227-1229 (1986).

Despite their therapeutic benefits, bisphosphonates are poorly absorbed from the gastrointestinal tract. *See* B.J. Gertz et al., *Clinical Pharmacology of Alendronate Sodium*, *Osteoporosis Int*., Suppl. 3: S13-16 (1993) and B.J. Gertz et al., *Studies of the oral bioavailability of alendronate*, *Clinical Pharmacology & Therapeutics,* vol. 58, number 3, pp. 288-298 (September 1995). Intravenous administration has been used to overcome this bioavailability problem. However, intravenous administration is costly and inconvenient, especially when the patient must be given an intravenous infusion lasting several hours on repeated occasions.

If oral administration of the bisphosphonate is desired, relatively high doses must be administered to compensate for the low bioavailability from the gastrointestinal tract. To offset this low bioavailability, it is generally recommended that the patient take the bisphosphonate on an empty stomach and fast for at least 30 minutes afterwards. However, many patients find the need for such fasting on a daily basis to be inconvenient. Moreover, oral administration has been associated with adverse gastrointestinal effects, especially those relating to the esophagus. *See* Fleisch, Id. These effects appear to be related to the irritant potential of the bisphosphonate in the esophagus, a problem which is exacerbated by the presence of refluxed gastric acid. For example, the bisphosphonate, pamidronate has been associated with esophageal ulcers. *See* E.G. Lufkin et al., *Pamidronate: An Unrecognized Problem in Gastrointestinal Tolerability, Osteoporosis International,* 4: 320-322 (1994). Although not as common, the use of alendronate has been associated with esophagitis and/or esophageal ulcers. *See* P.C. De Groen, et al., *Esophagitis Associated With The Use Of Alendronate*, *New England Journal of Medicine,* vol. 335, no. 124, pp. 1016-1021 (1996), D.O. Castell, *Pill Esophagitis -- The Case of Alendronate, New England Journal of Medicine,* vol. 335, no. 124, pp. 1058-1059 (1996), and U.A. Liberman et al., *Esophagitis and Alendronate*, *New England Journal of Medicine,* vol. 335, no. 124, pp. 1069-1070 (1996). The degree of adverse gastrointestinal effects of bisphosphonates has been shown to increase with increasing dose. *See* C.H. Chestnut et al., *Alendronate Treatment of the Postmenopausal Osteoporotic Woman: Effect of Multiple Dosages on Bone Mass and Bone Remodeling*, *The American Journal of Medicine,* vol. 99, pp. 144-152, (August 1995). Also, these adverse esophageal effects appear to be more prevalent in patients who do not take the bisphosphonate with an adequate amount of liquid or who lie down shortly after dosing, thereby increasing the chance for esophageal reflux.

Current oral bisphosphonate therapies generally fall into two categories: (1) those therapies utilizing continuous daily treatment, and (2) those therapies utilizing a cyclic regimen of treatment and rest periods.

The continuous daily treatment regimens normally involve the chronic administration of relatively low doses of the bisphosphonate compound, with the objective of delivering the desired cumulative therapeutic dose over the course of the treatment period. However, continuous daily dosing has the potential disadvantage of causing adverse gastrointestinal effects due to the repetitive, continuous; and additive irritation to the gastrointestinal tract. Also, because bisphosphonates should be taken on an empty stomach followed by fasting and maintenance of an upright posture for at least 30 minutes, many patients find daily dosing to be burdensome. These factors can therefore interfere with patient compliance, and in severe cases even require cessation of treatment.

Cyclic treatment regimens were developed because some bisphosphonates, such as etidronate, when given daily for more than several days, have the disadvantage of actually causing a decline in bone mineralization, i.e. osteomalacia. U.S. Patent No. 4,761,406, to Flora et al, issued August 2, 1988 describes a cyclic regimen developed in an attempt to minimize the decline in bone mineralization while still providing a therapeutic anti-resorptive effect. Generally, cyclic regimens are characterized as being intermittent, as opposed to continuous treatment regimens, and have both treatment periods during which the bisphosphonate is administered and nontreatment periods to permit the systemic level of the bisphosphonate to return to baseline. However, the cyclic regimens, relative to continuous dosing, appear to result in a decreased therapeutic antiresorptive efficacy. Data on risedronate suggests that cyclic dosing is actually less effective than continuous daily dosing for maximizing antiresorptive bone effects. *See L.* Mortensen, et al., *Prevention Of Early Postmenopausal Bone Loss By Risedronate*, *Journal of Bone and Mineral Research,* vol. 10, supp. 1, p. s140 (1995). Furthermore, these cyclic regimens do not eliminate or minimize adverse gastrointestinal effects, because such regimens typically utilize periods of multiple daily dosing. Also, the cyclic regimens are cumbersome to administer and have the disadvantage of low patient compliance, and consequently compromised therapeutic efficacy. U.S. Patent No. 5,366,965, to Strein, issued November 22, 1994, attempts to address the problem of adverse gastrointestinal effects by administering a polyphosphonate compound, either orally, subcutaneously, or intravenously, according to an intermittent dosing schedule having both a bone resorption inhibition period and a no-treatment rest period. However, the regimen has the disadvantage of not being continuous and regular, and requires nontreatment periods ranging from 20 to 120 days. PCT Application No. WO 95/30421, to Goodship et al, published November 16, 1995, discloses methods for preventing prosthetic loosening and migration using various bisphosphonate compounds. Administration of a once weekly partial dose of the bisphosphonate is disclosed. However, the reference specifically fails to address the issue of adverse gastrointestinal effects or to disclose administration of larger or multiple dosages.

Lunar News, July 1996, Update: Bisphosphonate, 23-24, discloses that, [t]he difficulties with oral bisphosphonates may favour their episodic (once/week), or cyclical (one week each month) administration. Even oral alendronate potentially could be given in a 40 or 80mg dose once/week to avoid dosing problems and reduce costs. Intravenous administration also is possible.'

Lunar News, April 1997, Update: Bisphosphonate, 30-32, discloses, 'One way to reduce the relatively high cost, and the potential side-effects, of potent oral bisphosphonates may be to dose only two or three times a week rather than daily... In the human, there is little difference between 5 and 10mg/day of alendronate, so the 10mg dose theoretically could be given only 3X a week or the 40mg dose once per week... If turnover remained low after three months at 3X/week, the dose could be reduced to 2X/week or lower.'

It is seen from current teachings that both daily and cyclic treatment regimens have shortcomings, and that there is a need for development of a dosing regimen to overcome these shortcomings.

In the present invention, it is found that the adverse gastrointestinal effects that can be associated with daily or cyclic dosing regimens can be minimized by administering the bisphosphonate at a relatively high unit dosage according to a continuous schedule having a dosing interval selected from the group consisting of once-weekly dosing. In other words, it is found that the administration of a bisphosphonate at a high relative dosage at a low relative dosing frequency causes less adverse gastrointestinal effects, particularly esophageal effects, compared to the administration of a low relative dosage at a high relative dosing frequency. This result is surprising in view of the teachings suggesting that adverse gastrointestinal effects would be expected to increase as a function of increasing bisphosphonate dosage. Such administration methods would be especially beneficial in treating patients that have been identified as suffering from or are susceptible to upper gastrointestinal disorders, e.g. gastrointestinal reflux disease (i.e. "GERD"), esophagitis, dyspepsia (i.e. heatburn), ulcers, and other related disorders. In such patients conventional bisphosphonate therapy could potentially exacerbate or induce such upper gastrointestinal disorders.

From a patient lifestyle standpoint, such methods would also be more convenient than daily or cyclic dosing regimens. Patients would be subjected less frequently to the inconvenience of having to take the drug on an empty stomach and having to fast for at least 30 minutes after dosing. Also patients would not need to keep track of a complex dosing regimen. Such methods are likely to have the advantage of promoting better patient compliance, which in turn can translate into better therapeutic efficacy.

It is an object of the present invention to provide uses for the manufacture of a medicament for inhibiting bone resorption and the conditions associated therewith.

It is another object of the present invention to describe methods which are oral methods.

It is another object of the present invention to describe such methods in humans.

It is another object of the present invention to describe such methods in patients that have been identified as suffering from or are susceptible to upper gastrointestinal disorders e.g. gastrointestinal reflux disease (i.e" GERD"), esophagitis, dyspepsia (i.e. heatburn), ulcers, and other related disorders.

It is another object of the present invention to describe such methods while minimizing the occurrence of or potential for adverse gastrointestinal effects.

It is another object of the present invention to describe such methods comprising a. continuous dosing schedule having a dosing interval of weekly dosing.

It is another object of the present invention to describe such methods wherein the continuous dosing schedule is maintained until the desired therapeutic effect is achieved.

These and other objects will become readily apparent from the detailed description which follows.

### SUMMARY OF THE INVENTION

The present invention relates to the use of alendronate in the manufacture of a medicament for treating osteoporosis in a human in need of such treatment, where said medicament is orally administered to said human as a unit dosage comprising about 70mg of the alendronate compound, on an alendronic acid active weight basis, according to a continuous schedule having a once-weekly dosing interval.

In other embodiments, the present invention describes such methods useful in humans identified as having or being susceptible to upper gastrointestinal disorders.

All percentages and ratios used herein, unless otherwise indicated, are by weight. The invention hereof can comprise, consist of, or consist essentially of the essential as well as optional ingredients, components, and methods described herein.

### BRIEF DESCRIPTION OF THE FIGURES

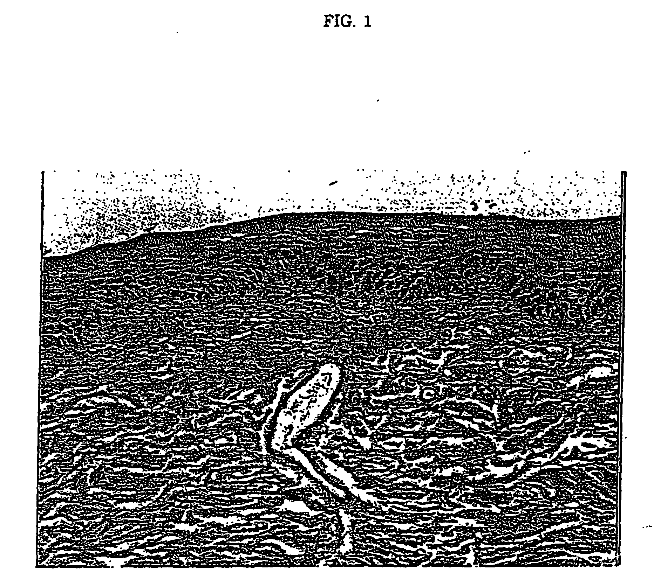

FIG. 1 is a photomicrograph (total magnification 270X) of canine esophagus tissue (paraffin embedded and stained with hematoxylin and eosin) from an animal sacrificed immediately after infusion of the last of five separate dosages of 50 mL of simulated gastric juice administered on five consecutive days.

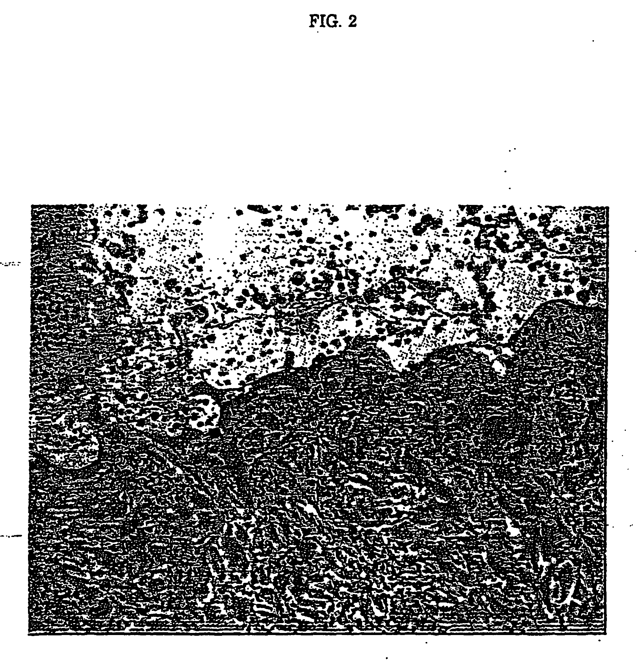

FIG. 2 is a photomicrograph (total magnification 270X) of canine esophagus tissue (paraffin embedded and stained with hematoxylin and eosin) from an animal sacrificed immediately after infusion of the last of five separate dosages of 50 mL of 0.20 mg/mL alendronate in simulated gastric juice administered on five consecutive days.

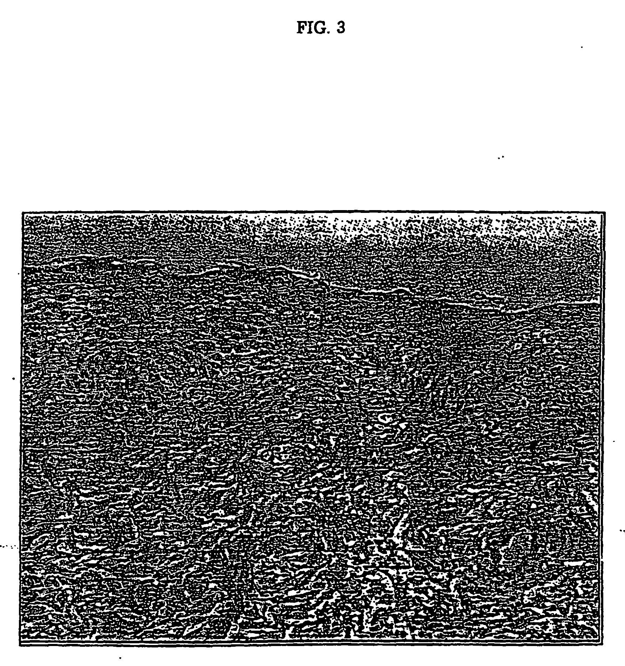

FIG. 3 is a photomicrograph (total magnification 270X) of canine esophagus tissue (paraffin embedded and stained with hematoxylin and eosin) from an animal sacrificed 24 hours after infusion with a single dosage of 50 mL of 0.80 mg/mL alendronate in simulated gastric juice.

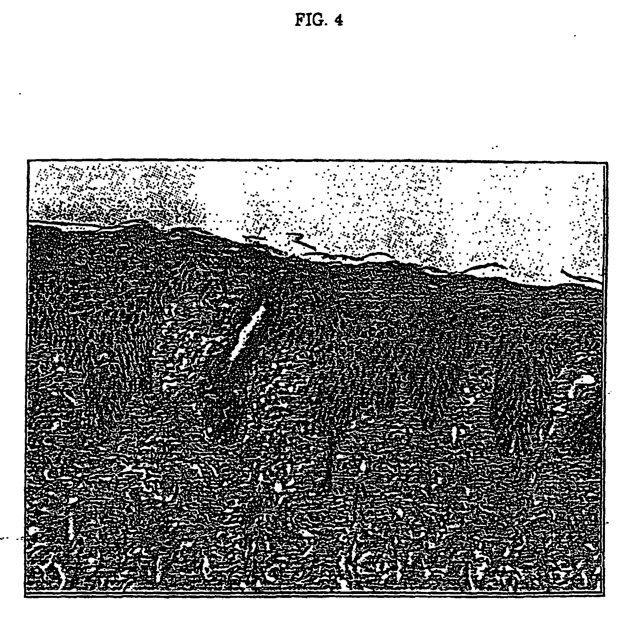

FIG. 4 is a photomicrograph (total magnification 270X) of canine esophagus tissue (paraffin embedded and stained with hematoxylin and eosin) from an animal sacrificed 7 days after infusion with a single dosage of 50 mL of 0.80 mg/mL alendronate in simulated gastric juice.

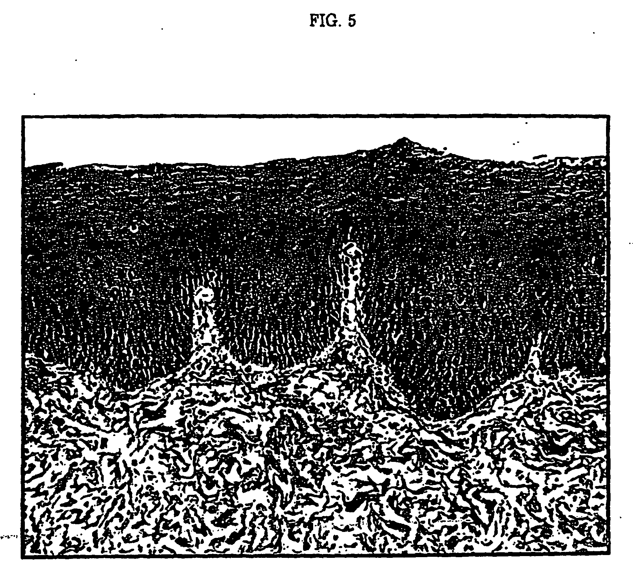

FIG. 5 is a photomicrograph (total magnification 270X) of canine esophagus tissue (paraffin embedded and stained with hematoxylin and eosin) from an animal sacrified 7 days after infusion of the last of 4 separate dosages of 50 mL of 0.80 mg/mL alendronate in simulated gastric juice administered once per week, i.e. once every 7 days.

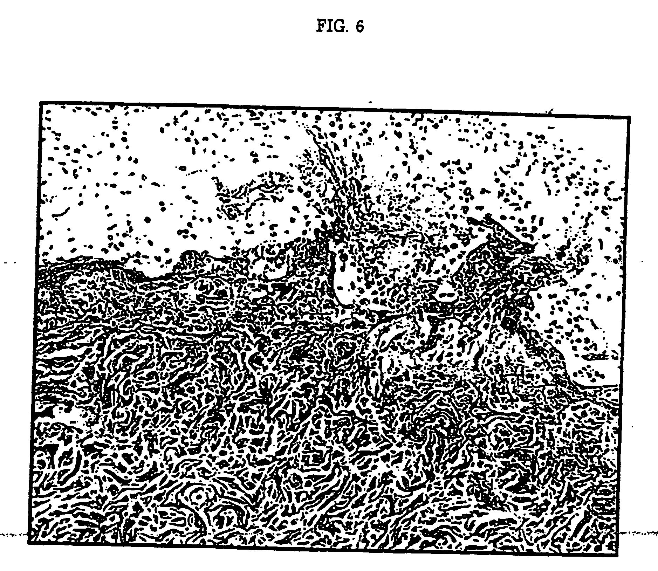

FIG. 6 is a photomicrograph (total magnification 270X) of canine esophagus tissue (paraffin embedded and stained with hematoxylin and eosin) from an animal sacrified 4 days after infusion of the last of 8 separate dosages of 50 mL of 0.40 mg/mL alendronate in simulated gastric juice administered twice per week, i.e. once every 3-4 days.

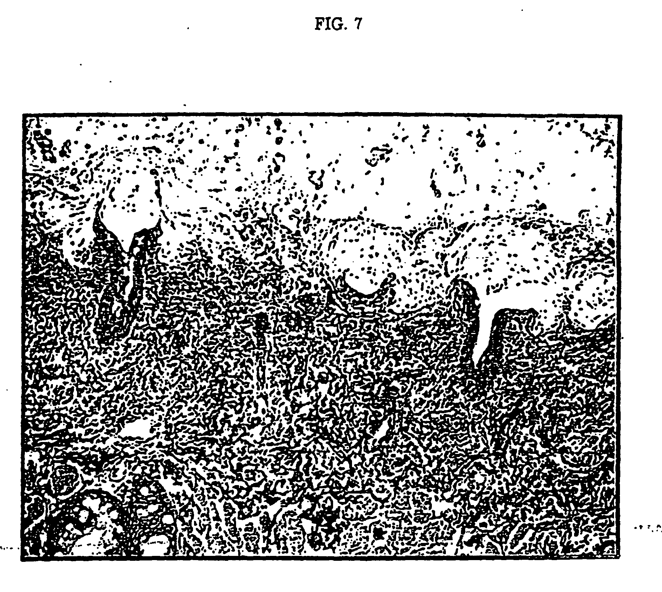

FIG. 7 is a photomicrograph (total magnification 270X) of canine esophagus tissue (paraffin embedded and stained with hematoxylin and eosin) from an animal sacrificed immediately after infusion of the last of five separate dosages of 50 mL of 0.20 mg/mL risedronate in simulated gastric juice administered on five consecutive days.

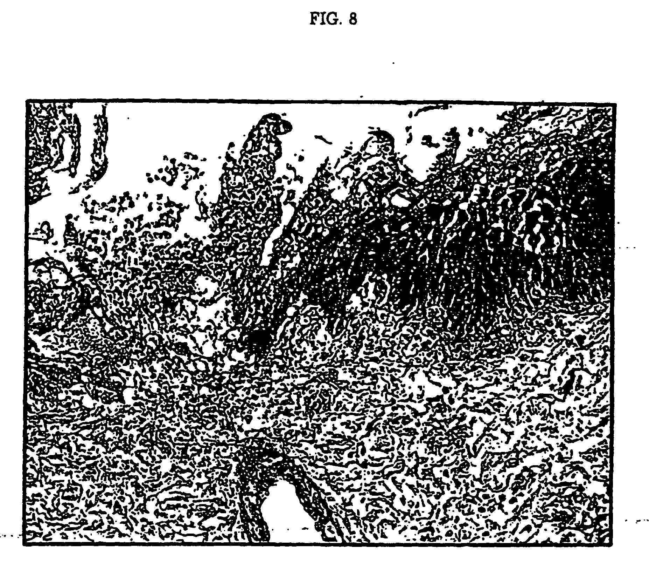

FIG. 8 is a photomicrograph (total magnification 270X) of canine esophagus tissue (paraffin embedded and stained with hematoxylin and eosin) from an animal sacrificed immediately after infusion of the last of five separate dosages of 50 mL of 4.0 mg/mL tiludronate in simulated gastric juice administered on five consecutive days.

### DESCRIPTION OF THE INVENTION

The present invention utilizes higher unit dosages of the bisphosphonate at each dosing point than has heretofore been typically administered, yet because of the dosing schedule chosen, the potential for adverse gastrointestinal effects are minimized. Moreover, the method is more convenient because the disadvantages associated with daily dosing are minimized.

The administration methods related to the present invention are especially useful in administering bisphosphonate therapy to human patients that have been identified as suffering from or are susceptible to upper gastrointestinal disorders, e.g. GERD, esophagitis, dyspepsia, ulcers, etc. In such patients conventional bisphosphonate therapy could potentially exacerbate or induce such upper gastrointestinal disorders.

The term "pharmaceutically effective amount", as used herein, means that amount of the bisphosphonate compound, that will elicit the desired therapeutic effect or response when administered in accordance with the desired treatment regimen.

The term "minimize the occurrence of or potential for adverse gastrointestinal effects", as used herein, means reducing, preventing, decreasing, or lessening the occurrence of or the potential for incurring unwanted side effects in the gastrointestinal tract, i.e. the esophagus, stomach, intestines, and rectum, particularly the upper gastrointestinal tract, i.e. the esophagus and stomach. Nonlimiting adverse gastrointestinal effects include, but are not limited to GERD, esophagitis, dyspepsia, ulcers, esophageal irritation, esophageal perforation, abdominal pain, and constipation.

The term "abnormal bone resorption", as used herein means a degree of bone resorption that exceeds the degree of bone formation, either locally, or in the skeleton as a whole. Alternatively, "abnormal bone resorption" can be associated with the formation of bone having an abnormal structure.

The term "bone resorption inhibiting", as used herein, means treating or preventing bone resorption by the direct or indirect alteration of osteoclast formation or activity. Inhibition of bone resorption refers to treatment or prevention of bone loss, especially the inhibition of removal of existing bone either from the mineral phase and/or the organic matrix phase, through direct or indirect alteration of osteoclast formation or activity.

The terms "continuous schedule" or "continuous dosing schedule", as used herein, mean that the dosing regimen is repeated until the desired therapeutic effect is achieved. The continuous schedule or continuous dosing schedule is distinguished from cyclical or intermittent administration.

The term "until the desired therapeutic effect is achieved", as used herein, means that the bisphosphonate compound is continuously administered, according to the dosing schedule chosen, up to the time that the clinical or medical effect sought for the disease or condition is observed by the clinician or researcher. For methods of treatment, the bisphosphonate compound is continuously administered until the desired change in bone mass or structure is observed. In such instances, achieving an increase in bone mass or a replacement of abnormal bone structure with more normal bone structure are the desired objectives. For methods of prevention of the present invention, the bisphosphonate compound is continuously administered for as long as necessary to prevent the undesired condition. In such instances, maintenance of bone mass density is often the objective. Nonlimiting examples of administration periods can range from about 2 weeks to the remaining lifespan of the mammal. For humans, administration periods can range from about 2 weeks to the remaining lifespan of the human, preferably from about 2 weeks to about 20 years, more preferably from about 1 month to about 20 years, more preferably from about 6 months to about 10 years, and most preferably from about 1 year to about 10 years.

The present invention does not have the disadvantages of current methods of treatment which can cause or increase the potential for adverse gastrointestinal effects or which require cumbersome, irregular, or complicated dosing regimens.

The present invention comprises a continuous dosing schedule whereby a unit dosage of the bisphosphonate is regularly administered according to a dosing interval.

By once-weekly dosing is meant that a unit dosage of the bisphosphonate is administered once a week, i.e. one time during a seven day period, preferably on the same day of each week. In the once-weekly dosing regimen, the unit dosage is generally administered about every seven days. A nonlimiting example of a once-weekly dosing regimen would entail the administration of a unit dosage of the bisphosphonate every Sunday. It is preferred that the unit dosage is not administered on consecutive days, but the once-weekly dosing regimen can include a dosing regimen in which unit dosages are administered on two consecutive days falling within two different weekly periods. The term "generalized bone loss" means bone loss at multiple skeletal sites or throughout the skeletal system. The term "localized bone loss" means bone loss at one or more specific, defined skeletal sites.

Generalized boss loss is often associated with osteoporosis. Osteoporosis is most common in post-menopausal women, wherein estrogen production has been greatly diminished. However, osteoporosis can also be steroid-induced and has been observed in males due to age. Osteoporosis can be induced by disease, e.g. rheumatoid arthritis, it can be induced by secondary causes, e.g., glucocorticoid therapy, or it can come about with no identifiable cause, i.e. idiopathic osteoporosis. In the present invention, preferred methods include the treatment or prevention of abnormal bone resorption in osteoporotic humans.

Localized bone loss has been associated with periodontal disease, with bone fractures, and with periprosthetic osteolysis (in other words where bone resorption has occured in proximity to a prosthetic implant).

Generalized or localized bone loss can occur from disuse, which is often a problem for those confined to a bed or a wheelchair, or for those who have an immobilized limb set in a cast or in traction.

The present invention is useful for treating the following conditions or disease states: osteoporosis, which can include post-menopausal osteoporosis, steroid-induced osteoporosis, male osteoporosis, disease-induced osteoporosis and idiopathic osteoporosis.

The methods related to the present invention are intended to specifically exclude methods for the treatment and/or prevention of prosthesis loosening and prosthesis migration in mammals as described in PCT application WO 95/30421, to Goodship et al, published November 16, 1995, which is incorporated by reference herein in its entirety.

### Bisphosphonates

The methods and compositions of the present invention comprise a bisphosphonate. The bisphosphonates of the present invention correspond to the chemical formula 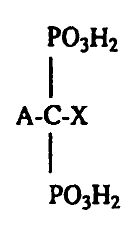 wherein
A is OH and X is a 3-aminopropyl moiety, so that the resulting compound is a 4-amino-1,-hydroxybutylidene-1,1-bisphosphonate, i.e. alendronate.

Pharmaceutically acceptable salts and derivatives of the bisphosphonates are also useful herein. Nonlimiting examples of salts include those selected from the group consisting alkali metal; alkaline metal, ammonium, and mono-, di, tri-, or tetra-C1-C30-alkyl-substituted ammonium. Preferred salts are those selected from the group consisting of sodium, potassium, calcium, magnesium, and ammonium salts. Nonlimiting examples of derivatives include those selected from the group consisting of esters, hydrates, and amides.

"Pharmaceutically acceptable" as used herein means that the salts and derivatives of the bisphosphonates have the same general pharmacological properties as the free acid form from which they are derived and are acceptable from a toxicity viewpoint.

It should be noted that the terms "bisphosphonate" and "bisphosphonates", as used herein in referring to the therapeutic agents of the present invention are meant to also encompass diphosphonates, biphosphonic acids, and diphosphonic acids, as well as salts and derivatives of these materials. The use of a specific nomenclature in referring to the bisphosphonate or bisphosphonates is not meant to limit the scope of the present invention, unless specifically indicated. Because of the mixed nomenclature currently in use by those or ordinary skill in the art, reference to a specific weight or percentage of a bisphosphonate compound in the present invention is on an acid active weight basis, unless indicated otherwise herein. For example, the phrase "about 70 mg of a bone resorption inhibiting bisphosphonate selected from the group consisting of alendronate, pharmaceutically acceptable salts thereof, and mixtures thereof, on an alendronic acid active weight basis" means that the amount of the bisphosphonate compound selected is calculated based on 70 mg of alendronic acid.

Nonlimiting examples of bisphosphonates useful herein include the following:
Alendronic acid, 4-amino-1-hydroxybutylidene-1,1-bisphosphonic acid.
Alendronate (also known as alendronate sodium or monosodium trihydrate), 4-amino-1-hydroxybutylidene-1,1-bisphosphonic acid monosodium trihydrate.
Alendronic acid and alendronate are described in U.S. Patents 4,922,007, to Kieczykowski et al., issued May 1, 1990, and 5,019,651, to Kieczykowski, issued May 28, 1991.

More preferred is alendronate, pharmaceutically acceptable salts thereof, and mixtures thereof.

Most preferred is alendronate monosodium trihydrate.

### Pharmaceutical Compositions

Compositions useful in the present invention comprise a pharmaceutically effective amount of a bisphosphonate. The bisphosphonate is typically administered in admixture with suitable pharmaceutical diluents, excipients, or carriers, collectively referred to herein as "carrier materials", suitably selected with respect to oral administration, i.e. tablets, capsules, elixirs, syrups, effervescent compositions, powders, and the like, and consistent with conventional pharmaceutical practices. For example, for oral administration in the form of a tablet, capsule, or powder, the active ingredient can be combined with an oral, non-toxic, pharmaceutically acceptable inert carrier such as lactose, starch, sucrose, glucose, methyl cellulose, magnesium stearate, mannitol, sorbitol, croscarmellose sodium and the like; for oral administration in liquid form, e.g., elixirs and syrups, effervescent compositions, the oral drug components can be combined with any oral, non-toxic, pharmaceutically acceptable inert carrier such as ethanol, glycerol, water and the like. Moreover, when desired or necessary, suitable binders, lubricants, disintegrating agents, buffers, coatings, and coloring agents can also be incorporated. Suitable binders can include starch, gelatin, natural sugars such a glucose, anhydrous lactose, free-flow lactose, beta-lactose, and corn sweeteners, natural and synthetic gums, such as acacia, guar, tragacanth or sodium alginate, carboxymethyl cellulose, polyethylene glycol, waxes, and the like. Lubricants used in these dosage forms include sodium oleate, sodium stearate, magnesium stearate, sodium benzoate, sodium acetate, sodium chloride and the like. A particularly preferred tablet formulation for alendronate monosodium trihydrate is that described in U.S. Patent No. 5,358,941, to Bechard et al, issued October 25, 1994. The compounds used in the present method can also be coupled with soluble polymers as targetable drug carriers. Such polymers can include polyvinylpyrrolidone, pyran copolymer, polyhydroxylpropyl-methacrylamide, and the like.

For once-weekly dosing, an oral unit dosage comprises about 70 mg of the alendronate compound, on an alendronic acid active weight basis. A unit dosage which is useful for treating osteoporosis comprises about 70 mg of the alendronate compound.

Nonlimiting examples of oral compositions comprising alendronate, as well as other bisphosphonates, are illustrated in the Examples, below.

### Sequential Administration Of Histamine H2 Receptor Blockers And/Or Proton Pump Inhibitors With Bisphosphonates

In further embodiments, the uses and compositions related to the present invention can also comprise a histamine H2 receptor blocker (i.e. antagonist) and/or a proton pump inhibitor. Histamine H2 receptor blockers and proton pump inhibitors are well known therapeutic agents for increasing gastric pH. *See* L.J. Hixson, et al., *Current Trends in the Pharmacotherapy for Peptic Ulcer Disease, Arch*. *Intern. Med*., vol. 152, pp. 726-732 (April 1992).

It is found in the present invention that the sequential oral administration of a histamine H2 receptor blocker and/or a proton pump inhibitor, followed by a bisphosphonate can help to further minimize adverse gastrointestinal effects. In these embodiments, the histamine H2 receptor blocker and/or proton pump inhibitor is administered from about 30 minutes to about 24 hours prior to the administration of the bisphosphonate. In more preferred embodiments, the histamine H2 receptor blocker and/or proton pump inhibitor is administered from about 30 minutes to about 12 hours prior to the administration of the bisphosphonate.

The dosage of the histamine H2 receptor blocker and/or proton pump inhibitor will depend upon the particular compound selected and factors associated with the mammal to be treated, i.e. size, health, etc.

Nonlimiting examples of histamine H2 receptor blockers and/or proton pump inhibitors include those selected from the group consisting of cimetidine, famotidine, nizatidine, ranitidine, omprazole, and lansoprazole.

### Treatment Kits

There is also disclosed a kit for conveniently and effectively carrying out the methods in accordance with the present invention. Such kits are especially suited for the delivery of solid oral forms such as tablets or capsules. Such a kit preferably includes a number of unit dosages. Such kits can include a card having the dosages oriented in the order of their intended use. An example of such a kit is a "blister pack". Blister packs are well known in the packaging industry and are widely used for packaging pharmaceutical unit dosage forms. If desired, a memory aid can be provided, for example in the form of numbers, letters, or other markings or with a calendar insert, designating the days in the treatment schedule in which the dosages can be administered. Alternatively, placebo dosages, or calcium or dietary supplements, either in a form similar to or distinct from the bisphosphonate dosages, can be included to provide a kit in which a dosage is taken every day. In those embodiments including a histamine H2 receptor and/or proton pump inhibitor, these agents can be included as part of the kit.

### EXAMPLES

The following examples further describe and demonstrate embodiments within the scope of the present invention.

### EXAMPLE 1

### Esophageal Irritation Potential

The esophageal irritation potential of the bisphosphonates is evaluated using a dog model.

The experiments demonstrate the relative irritation potential of the following dosing regimens: placebo (Group 1), a single high concentration dosage of alendronate monosodium trihydrate (Group 2), a low concentration dosage of alendronate monosodium trihydrate administered for five consecutive days (Groups 3 and 4), a high concentration dosage of alendronate monosodium trihydrate administered once per week for four weeks (Group 5), a mid-range concentration dosage of alendronate monosodium trihydrate administered twice per week for four weeks (Group 6), a low dosage of risedronate sodium administered for five consecutive days (Group 7), and a low dosage of tiludronate disodium administered for five consecutive days (Group 8).

The following solutions are prepared:
(1) simulated gastric juice (pH about 2), i.e. the control solution.
(2) simulated gastric juice (pH about 2) containing about 0.20 mg/mL of alendronate monosodium trihydrate on an alendronic acid active basis.
(3) simulated gastric juice (pH about 2) containing about 0.80 mg/mL of alendronate monosodium trihydrate on an alendronic acid active basis.
(4) simulated gastric juice (pH about 2) containing about 0.40 mg/mL of alendronate monosodium trihydrate on an alendronic acid active basis.
(5) simulated gastric juice (pH about 2) containing about 0.20 mg/mL of risedronate sodium on a risedronic acid active basis.
(6) simulated gastric juice (pH about 2) containing about 4.0 mg/mL of tiludronate disodium on a tiludronic acid active basis.
The simulated gastric juice is prepared by dissolving about 960 mg of pepsin (L-585,228000B003, Fisher Chemical) in about 147 mL of 0.90 (wt %) NaCl (aqueous), adding about 3mL of 1.0 M HCl (aqueous), and adjusting the volume to about 300 mL with deionized water. The pH of the resulting solution is measured and if necessary is adjusted to about 2 using 1.0 M HCl (aqueous) or 1.0 M NaOH (aqueous).

The animals used in the experiments are anesthetized and administered about 50 mL of the appropriate solution over about 30 minutes by infusion into the esophagus using an infusion pump and a rubber catheter. The following treatment experiments are run:
Group 1: This control group contains four animals. Each animal is administered a dosage of about 50 mL of simulated gastric juice [solution (1)] on each of five consecutive days. The animals are sacrificed immediately after the last dose is administered.
Group 2: This group contains four animals. Each animal is administered a dosage of about 50 mL of simulated gastric juice containing about 0.20 mg/mL of alendronate [solution (2)] on each of five consecutive days. The animals are sacrificed immediately after the last dose is administered.
Group 3: This group contains five animals. Each animal is administered a dosage of about 50 mL of simulated gastric juice containing about 0.80 mg/mL of alendronate [solution (3)] on a single treatment day. The animals are sacrificed about 24 hours after the dose is administered.
Group 4: This group contains five animals. Each animal is administered a dosage of about 50 mL of simulated gastric juice containing about 0.80 mg/mL of alendronate [solution (3)] on a single treatment day. The animals are sacrificed about 7 days after the dose is administered.
Group 5: This group contains six animals. Each animal is administered a dosage of about 50 mL of simulated gastric juice containing about 0.80 mg/mL of alendronate [solution (3)] once per week, i.e. every seven days, for four weeks. The animals are administered a total of four dosages. The animals are sacrificed about 7 days after the last dose is administered.
Group 6: This group contains six animals. Each animal is administered a dosage of about 50 mL of simulated gastric juice containing about 0.40 mg/mL of alendronate [solution (4)] twice per week, i.e. every three to four days, for four weeks. The animals are administered a total of eight dosages. The animals are sacrificed about four days after the last dose is administered.
Group 7: This group contains eight animals. Each animal administered a dosage of about 50 mL of simulated gastric juice containing about 0.20 mg/mL of risedronate [solution (5)] on each of five consecutive days. The animals are sacrificed immediately after the last dose is administered.
Group 8: This group contains four animals. Each animal is administered a dosage of about 50 mL of simulated gastric juice containing about 4.0 mg/mL of tiludronate [solution (6)] on each of five consecutive days. The animals are sacrificed immediately after the last dose is administered.

The esophagus from each sacrificed animal is removed and prepared for histopathology using standard techniques by embedding the tissue in paraffin, staining with hematoxylin and eosin. The sections are examined microscopically. The histopathology results are summarized in Table 1.

For the Group 1 animals (control group), the photomicrographs show that the esophagus is normal with an intact epithelium and absence of inflammatory cells in the submucosa. FIG. 1 is a representative photomicrograph from a Group 1 animal.

For the Group 2 animals, the photomicrographs show that the esophagus exhibits deep ulceration of the epithelial surface and marked submucosal inflammation and vacuolation. FIG. 2 is a representative photomicrograph from a Group 2 animal.

For the Group 3 animals, the photomicrographs show that the esophagus has an intact epithelial surface with very slight submucosal inflammation and vacuolation. FIG. 3 is a representative photomicrograph from a Group 3 animal.

For the Group 4 animals, the photomicrographs show that the esosphagus has an intact epithelium with either minimal inflammation (two of the five animals) or no inflammation (three of the five animals) and no vacuolation. FIG. 4 is a representative photomicrograph from a Group 4 animal exhibiting minimal inflammation.

For the Group 5 animals, the photomicrographs show that the esophagus is normal with an intact epithelium and absence of inflammatory cells in the submucosa. FIG. 5 is a representative photomicrograph from a Group 5 animal.

For the Group 6 animals, the photomicrographs show that the esophagus exhibits deep ulceration of the epithelial surface and marked submucosal inflammation and vacuolation. FIG. 6 is a representative photomicrograph from a Group 6 animal.

For the Group 7 animals, the photomicrographs show that the esophagus exhibits deep ulceration of the epithelial surface and marked submucosal inflammation and vacuolation. FIG. 7 is a representative photomicrograph from a Group 7 animal.

For the Group 8 animals, the photomicrographs show that the esophagus exhibits slight ulceration of the epithelial surface and slight submucosal inflammation and vacuolation. FIG. 8 is a representative photomicrograph from a Group 8 animal.

These experiments demonstrate that considerably less esophageal irritation (comparable to control Group 1)is observed from the administration of a single high concentration dosage of alendronate (Groups 3 and 4) versus administration of low concentration dosages on consecutive days (Group 2). These experiments also demonstrate consideraly less esophageal irritation is observed from the administration of a single high concentration of alendronate on a weekly basis (Group 5) or twice-weekly basis (Group 6) versus administration of low concentration dosages on consecutive days (Group 2). These experiments also demonstrate that when other bisphosphonates such as risedronate (Group 7) or tiludronate (Group 8) are administered at low dosages on consecutive days that the esophageal irritation potential is high.

**Table 1.**

| **Esophageal Irritation Potential Studies** | | | | |
|---|---|---|---|---|
| **Group** | **Active Agent mg/mL** | **Dosing Schedule** | **Sacrifice Time** | **Histo-pathology** |
| 1 | 0 | 1X daily | immediate | Normal. Intact |
| (n=4) | | for 5 days | ly after last dosing | epithelium and absence of inflammatory cells in the submucosa. |
| 2 | Alendronate | 1X daily | immediate | Deep ulceration of |
| (n=4) | 0.20 | for 5 days | ly after last dosing | epithelial surface. Marked submucosal inflammation and vacuolation. |
| 3 | Alendronate | 1X | 24 hours | Intact epithelial |
| (n=5) | 0.80 | | after dosing | surface with very slight submucosal inflammation and vacuolation. |
| 4 | Alendronate | 1X | 7 days | Intact epithelium |
| (n=5) | 0.80 | | after dosing | with either minimal inflammation (2 of 5 animals) or no inflammation (3 of 5 animals) and no vacuolation. |
| 5 | Alendronate | 1X | 7 days | Intact epithelium |
| (n=6) | 0.80 | weekly for a total of 4 doses | after last dosing | with no inflammation and no vacuolation. |
| 6 | Alendronate | 2X | immediate | Deep ulceration of |
| (n=6) | 0.40 | weekly for 4 weeks | ly after last dosing | epithelial surface. Marked submucosal inflammation and vacuolation. |
| 7 | Risedronate | 1X daily | immediate | Deep ulceration of |
| (n=8) | 0.20 | for 5 days | ly after last dosing | epithelial surface (4 of 8 animals). Marked submucosal inflammation and vacuolation. |
| 8 | Tiludronate | 1X daily | 24 hours | Slight submucosal |
| (n=4) | 4.0 | for 5 days | after last dosing | inflammation and vacuolation (3 of 4 animals, including 1 of these animals with slight ulceration). |

### EXAMPLE 2

### Once-weekly dosing regimen.

### Treatment of osteoporosis.

Alendronate tablets or liquid formulations containing about 70 mg of alendronate, on an alendronic acid active basis, are prepared (see EXAMPLES 7 and 8). The tablets or liquid formulations are orally administered to a human patient once-weekly, i.e. preferably about once every seven days (for example, every Sunday), for a period of at least one year. This method of administration is useful and convenient for treating osteoporosis and for minimizing adverse gastrointestinal effects, particularly adverse esophageal effects. This method is also useful for improving patient acceptance and compliance.

### Prevention of osteoporosis.

Alendronate tablets or liquid formulations containing about 35 mg of alendronate, on an alendronic acid active basis, are prepared (see EXAMPLES 3 and 4). The tablets or liquid formulations are orally administered to a human patient once-weekly, i.e. preferably about once every seven days (for example, every Sunday), for a period of at least one year. This method of administration is useful and convenient for preventing osteoporosis and for minimizing adverse gastrointestinal effects, particularly adverse esophageal effects. This method is also useful for improving patient acceptance and compliance.

### EXAMPLE 3

### Bisphosphonate tablets.

Bisphosphonate containing tablets are prepared using standard mixing and formation techniques as described in U.S. Patent No. 5,358,941, to Bechard et al., issued October 25,1994.

Tablets containing about 35 mg of alendronate (for reference), on an alendronic acid active basis, are prepared using the following relative weights of ingredients.

| Ingredient | Per Tablet | Per 4000 Tablets |
|---|---|---|
| Alendronate Monosodium Trihydrate | 45.68 mg | 182.72 g |
| Anhydrous Lactose, NF | 71.32 mg | 285.28 g |
| Microcrystalline Cellulose, NF | 80.0 mg | 320.0 g |
| Magnesium Stearate, NF | 1.0 mg | 4.0 g |
| Croscarmellose Sodium, NF | 2.0 mg | 8.0 g |

Similarly, tablets comprising other relative weights of alendronate, on an alendronic acid active basis are prepared: e.g., about 70 mg per tablet.

### EXAMPLE 4

### Liquid Bisphosphonate Formulation.

Liquid bisphosphonate formulations are prepared using standard mixing techniques.

A liquid formulation containing about 70 mg of alendronate monosodium trihydrate, on an alendronic acid active basis, per about 75 mL of liquid is prepared using the following relative weights of ingredients.

| Ingredient | Weight |
|---|---|
| Alendronate Monosodium Trihydrate | 91.35 mg |
| Sodium Propylparaben | 22.5 mg |
| Sodium Butylparaben | 7.5 mg |
| Sodium Citrate Dihydrate | 1500 mg |
| Citric Acid Anhydrous | 56.25 mg |
| Sodium Saccharin | 7.5 mg |
| Water | qs 75 mL |
| 1 N Sodium Hydroxide (aq) | qs pH 6.75 |

## Claims

1. Use of alendronate in the manufacture of a medicament for treating osteoporosis in a human in need of such treatment, where said medicament is orally administered to said human as a unit dosage comprising about 70mg of the alendronate compound, on an alendronic acid active weight basis, according to a continuous schedule having a once-weekly dosing interval.

2. A use according to claim 1 wherein the bisphosphonate is alendronate monosodium trihydrate.

3. A use according to claim 1 or 2 where the composition is a tablet, capsule or powder.

4. A use according to claim 1 or 2 where the composition is a tablet or capsule.

5. A use according to claim 1 or 2 where the composition is a tablet.

6. A use according to claim 1 or 2 where the composition is a capsule.

## Patentansprüche

1. Verwendung von Alendronat bei der Herstellung eines Medikaments zur Behandlung von Osteoporose bei einem Menschen, der eine solche Behandlung benötigt, wobei das Medikament an den Menschen als eine Einheitsdosis, die etwa 70 mg der Alendronatverbindung auf Gewichtsbasis an aktiver Alendronsäure enthält, gemäß einem Dauertherapiepian mit einem Dosierungsintervall von einmal pro Woche oral verabreicht wird.

2. Eine Verwendung gemäß Anspruch 1, wobei das Bisphosphonat Alendronat-Mononatrium-Trihydrat ist.

3. Eine Verwendung gemäß Anspruch 1 oder 2, wobei die Zusammensetzung eine Tablette, eine Kapsel oder ein Pulver ist.

4. Eine Verwendung gemäß Anspruch 1 oder 2, wobei die Zusammensetzung eine Tablette oder Kapsel ist.

5. Eine Verwendung gemäß Anspruch 1 oder 2, wobei die Zusammensetzung eine Tablette ist.

6. Eine Verwendung gemäß Anspruch 1 oder 2, wobei die Zusammensetzung eine Kapsel ist.

## Revendications

1. Utilisation d'alendronate dans la fabrication d'un médicament pour traiter l'ostéoporose chez l'être humain ayant besoin d'un tel traitement, où ledit médicament est administré par voie orale audit être humain sous forme d'un dosage unitaire comprenant environ 70 mg du composé alendronate, sur la base du poids actif en acide alendronique, selon un calendrier continu ayant un intervalle de dosage d'une fois par semaine.

2. Utilisation selon la revendication 1, dans laquelle le bisphosphonate est un trihydrate d' alendronate monosodique.

3. Utilisation selon la revendication 1 ou 2, où la composition est un comprimé, une capsule ou une poudre.

4. Utilisation selon la revendication 1 ou 2, où la composition est un comprimé ou une capsule.

5. Utilisation selon la revendication 1 ou 2, où la composition est un comprimé.

6. Utilisation selon la revendication 1 ou 2, où la composition est une capsule.
